# EUROPEAN PATENT APPLICATION

(11) **EP 3 305 289 A1**
(43) Date of publication of application: **11.04.2018**
(21) Application number: 16192542.5
(22) Date of filing: 06.10.2016
(51) Int. Cl.: A61K 31/357, A61P 31/12

(54) **USAGE OF SILVESTROL, EPISILVESTROL AND SILVESTROL ANALOGA FOR THE TREATMENT OF VIRAL INFECTIONS CAUSED BY VIRUSES WITH CAP-DEPENDENT TRANSLATION**

(71) Applicant: PHILIPPS-UNIVERSITÄT MARBURG, 35037 Marburg (DE); Justus-Liebig-Universität Gießen, 35390 Gießen (DE)
(72) Inventor: Grünweller, Arnold, 35094 Lahntal-Goßfelden (DE); Hartmann, Roland K., 35041 Marburg (DE); Lange-Grünweller, Kerstin, 35094 Lahntal-Goßfelden (DE); Schulte, Falk, 35041 Marburg (DE); Becker, Stephan, 35037 Marburg (DE); Biedenkopf, Nadine, 35392 Gießen (DE); Ziebuhr, John, 35614 Aßlar (DE); Müller, Christin, 35753 Greifenstein (DE); Schlitzer, Martin, 35043 Marburg (DE)
(74) Representative: Stumpf, Peter

(57) **Abstract**

The invention relates to the use of Silvestrol, Episilvestrol and Silvestrol/Episilvestrol analoga/derivatives for treating infections with viruses whose protein translation requires a cap-structure at the 5'-end of viral mRNAs, especially for treating infections with (-)RNA-strand viruses like the family of *Filoviridae* (Ebola- and Marburgvirus) as well as (+)RNA-strand viruses like the family of *Coronaviridae* (e.g. MERS and SARS).

## Description

### Field of the invention

The invention relates to the use of Silvestrol, Episilvestrol, Silvestrol analoga and other Rocaglates (also called Flavaglines) for treating infections with viruses whose protein translation requires a cap-structure at the 5'-end of viral mRNAs, especially for treating infections with (-)RNA-strand viruses with a negative-sense single-stranded RNA genome like the family of *Filoviridae* (Ebola- and Marburgvirus) as well as (+)RNA-strand viruses with a positive-sense single-stranded RNA genome like the family of *Coronaviridae* (e.g. MERS-CoV and SARS-CoV). The use of Silvestrol, Episilvestrol, Silvestrol analoga and other Rocaglates against other RNA viruses with cap-dependent translational initiation is also part of this invention.

### Background of the technology

Silvestrol, a natural compound isolated from the plant *Aglaia foveolata*, is a potent and specific inhibitor of the ATP-dependent DEAD-box RNA helicase eIF4A, which is part of the heterotrimeric translation initiation complex eIF4F in eukaryotes. It has been proposed that Silvestrol increases the affinity of eIF4A for the bound target mRNA, thereby stalling the helicase on its RNA substrate. Silvestrol exerts potent antitumor activity *in vitro* and *in vivo* by inhibiting translation of short-lived oncoproteins, such as c-MYC and PIM1, whose mRNA 5'-UTRs are extended and include regions of stable RNA structures that require unwinding by eIF4A to create a binding platform for the 43S preinitiation complex. Chu et al. (Chu et al. 2016, Cell Reports 15, 2340-2347) show that the anti-neoplastic activity of rocaglates is a consequence of the eIF4A1 inhibition and also found that a synthetic derivative is even more efficient than the natural compound. Therefore, eIF4A is discussed as a promising new drug target for cancer treatment.

The recent outbreak of the Ebola virus (EBOV) in West Africa caused more than 28,000 cases with at least 11,000 fatalities, revealing that effective viral inhibitors with potential broad-spectrum activity are urgently needed. EBOV is a negative-sense single-stranded RNA virus encoding seven genes. Transcription of the genes is accomplished by the viral polymerase complex resulting in monocistronic, 5' capped and 3' polyadenylated mRNAs. As for all viruses, EBOV protein synthesis depends on the cellular translation machinery. Interestingly, some EBOV mRNAs harbor long 5'-UTRs and all EBOV mRNAs were predicted or demonstrated to adopt stable RNA secondary structures in their 5'-UTRs. Thus efficient cap-dependent translation of EBOV mRNAs also requires the host helicase activity of eIF4A to unwind such structures during initiation of translation.

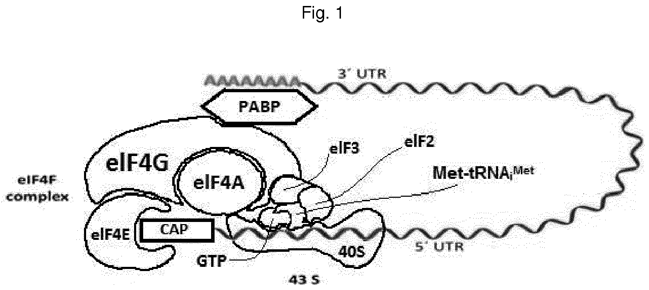

Silvestrol, as well as other rocaglates, exerts very low toxicity within normal cells and, due to its highly specific inhibition of the eIF4A RNA-helicase, a selective inhibition of the cap-dependent translation of proto-oncogenes (cf. Fig. 1).

Thus it is known in the state of the art to use Silvestrol (a derivative of rocaglate with a cyclopenta*[*b*]*benzofuran skeleton) for the treatment of cancer. It is not known in the state of the art to use Silvestrol or Silvestrol analoga (as well as Episilvestrol) for the treatment of virus infections. Moreover, in comparison to other known compounds with antiviral activity, Silvestrol, Episilvestrol and their analoga exert a very potent antiviral activity against Coronaviruses with a (+) RNA strand genome whose protein translation is also cap-dependent and at the same time has no specific antiviral activity against viruses whose protein translation can be initiated by an internal ribosome entry site (IRES) (for example Rhinovirus 1A and Poliovirus).

Silvestrol is generally inhibiting the cap-dependent translation of structured viral mRNAs. Therefore Silvestrol, Episilvestrol and their analogs and derivatives are effective as "broadband" virostatics. This is, because only if viruses can perform a cap-independent translation (e.g. Rhinoviruses and Polioviruses), there is no main dependency to cellular eIF4A-helicase, due to the fact that then the ribosomeas-sembly can take place directly at a IRES-sequence. Thus the unwinding of RNA secondary structures with the help of eIF4A in order to provide a binding-platform for the 43S preinitiation complex is not necessary in this case. It is well known to the person skilled in the art that this effect - so far found at the Ebola- and Coronaviruses (Coronaviridae like MERS-CoV) - may universally prevail with all types of viruses which translate cap-dependently, i.e. without IRES (e.g., Flaviviridae like the Zikavirus and the Denguevirus, Togaviridae like the Chikungunyavirus or the Semliki-Forest virus, Orthomyxoviridae like Influenza viruses-, Bunya-, Rhabdo-, Paramyxoviridae etc.). Thus it is obvious to the person skilled in the art that the scope of the invention is not confined to the Filoviridae (Ebola- and Marburgvirus), but comprises all types of viruses which also perform cap-dependent protein translation.

Because the inhibition of the cap-dependent translation is most effective during early states of viral infections, the application of Silvestrol, Episilvestrol and their derivatives and analogues is most helpful at acute infections with viruses that show short viral replication cycles.

The scope of the invention is even more obvious if one considers that most types of viruses can escape antiviral active principles that are aiming towards viral targets by use of so called escape-mutations. This strategy is very difficult with host cell targets that are also concerning vital processes for viral growth like the eIF4A-helicase, for any mutation of eIF4A will most likely also impair vital processes of virus proliferation.

Regarding Coronaviruses Silvestrol shows an extraordinary high efficacy with EC₅₀-values of 1-3 nM. Cell cytotoxicity is with CC₅₀-values of about 2 µM about 1000 times higher, leading to a selectivity index of about 1000. This is an extremely high value, showing that Silvestrol, Episilvestrol and their analogs and derivatives offer a very high antiviral specifity.

### Content of the invention

### Silvestrol inhibits EBOV propagation in Huh-7 cells

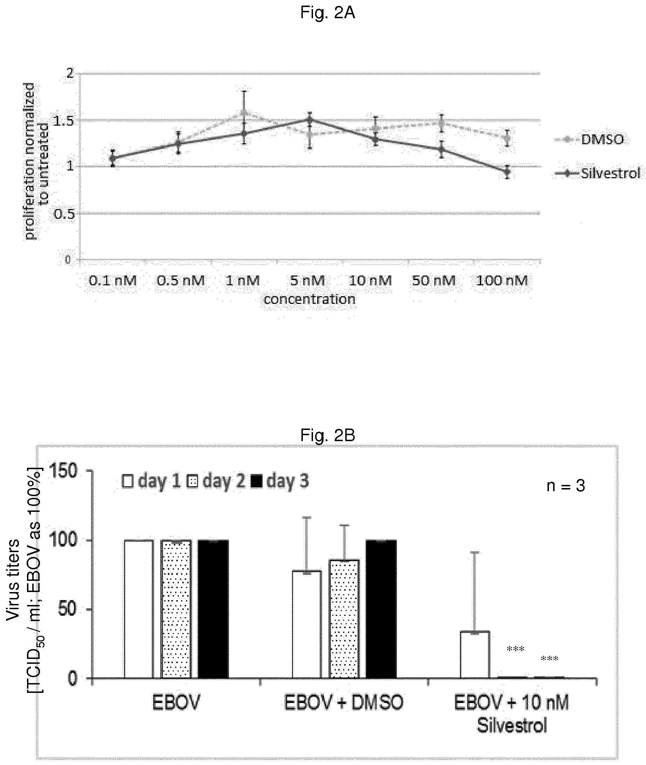

The effects of Silvestrol on proliferation of Huh-7 cells, a standard hepatoma cell line to investigate EBOV is analyzed by using Silvestrol concentrations in a range between 0.1 nM - 100 nM. Up to 10 nM Silvestrol no obvious effects on proliferation could be detected. At higher concentrations cell proliferation is weakly affected compared to the DMSO control (Fig. 2A).

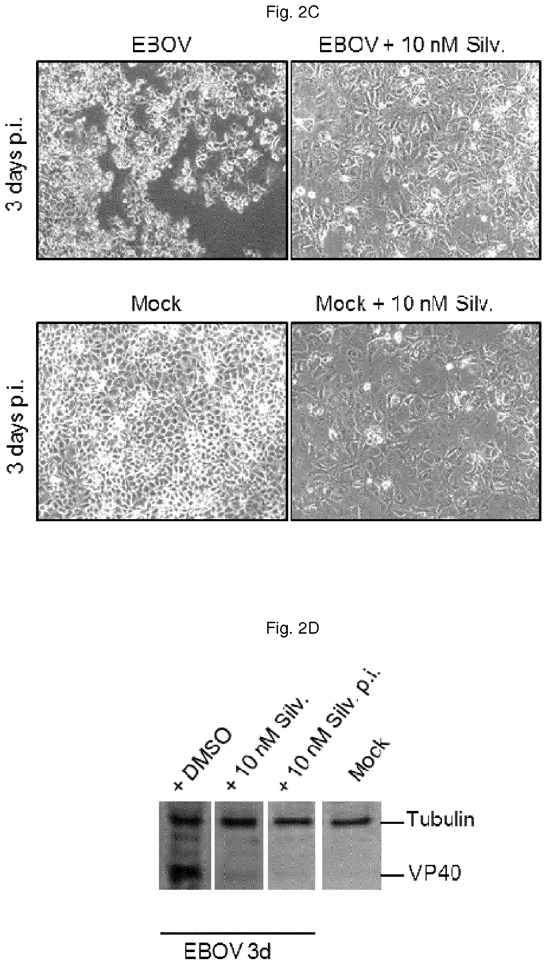

For testing the potential antiviral activity of Silvestrol, Huh-7 cells are preincubated for 2 h with 10 nM Silvestrol and then infected with EBOV at a multiplicity of infection (MOI) of 0.1. Viral titers of supernatants were determined by TCID₅₀ analysis in VeroE6 cells at day 1, 2, and 3 (p.i.). Remarkably, a concentration of 10 nM Silvestrol substantially inhibits EBOV infection (Fig. 2B) without changing cell morphology as determined by light microscopy (Fig. 2C).

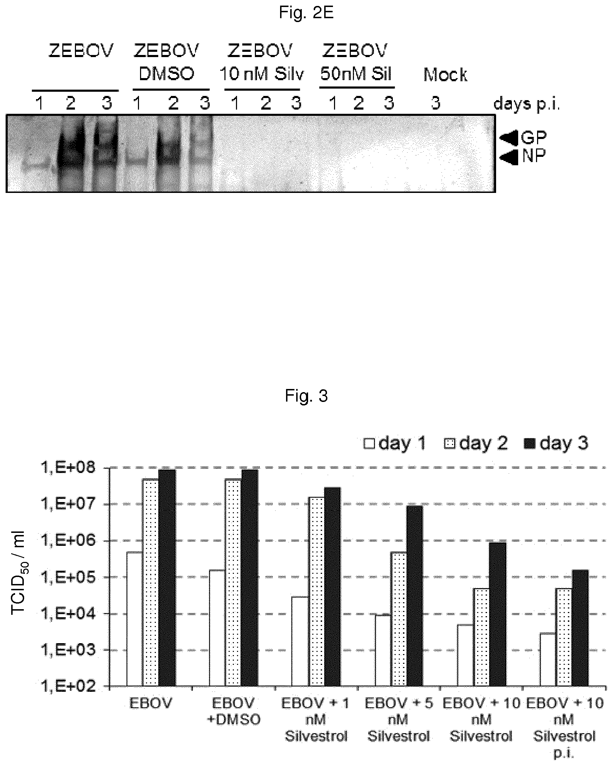

The specificity of Silvestrol on viral mRNA translation is shown via investigation of its impact on expression of the EBOV protein VP40 and the housekeeping protein tubulin by Westernblotting. A strong reduction of VP40 levels by treating cells with 10 nM Silvestrol 2 h before or post EBOV infection is taking place (Fig. 2D). Similar reduction in protein levels also does appear for the EBOV proteins NP and GP (Fig. 2E). These results clearly show a substantial antiviral effect of Silvestrol in EBOV-infected Huh-7 cells. It is obvious to a person skilled in the art that analogs and derivatives of Silvestrol and Episilvestrol may offer the same or even more intense antiviral effects and/or the same or even less cytotoxicity. Therefore the scope of the invention is not limited to Silvestrol, but also comprises analogs and derivatives of Silvestrol and Episilvestrol.

At a concentration of only 1 nM Silvestrol causes a reduction of the EBOV titer by almost one order of magnitude and at 5 nM the inhibitory effect of Silvestrol increases up to 1-2 log orders (Fig. 3). Again, treatment of Huh-7 cells with 10 nM Silvestrol inhibits viral propagation efficiently. A comparable antiviral effect can also be measured when Silvestrol treatment is started immediately after EBOV infection (p.i.) (Fig. 3, last panel).

### Silvestrol is not efficient against Rhinovirus 1A harbouring an IRES

Silvestrol can also inhibit the propagation of ssRNA viruses with a (+) RNA genome. The effects of Silvestrol in primary MRC-5 cells (derived from normal lung tissue) infected with human pathogenic Coronaviruses proves this.

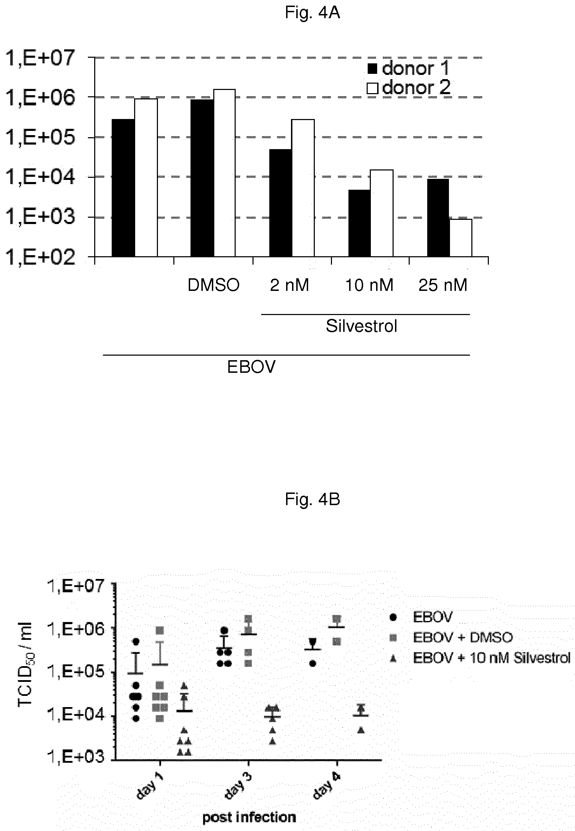

Beneath Cap-dependent translation several viruses, like Picornaviruses, can initiate translation via an IRES. Therefore the antiviral activity of Silvestrol in Rhinovirus 1A infected Hela cells was investigated, finding an EC₅₀ value of 400 nM, which is about 10-fold higher compared to Huh-7 infected HCoV-229E cells (EC₅₀ of 40 nM; see Fig. 4A-D). Since Silvestrol has potent antitumor activity in a large set of cancer cell lines, the observed reduction in virus titer in Rhinovirus 1A infected Hela cells at concentrations >100 nM should be mainly caused by antiproliferative effects of Silvestrol through the inhibition of proto-oncogenes and by induction of apoptosis. From this it is to be concluded that Silvestrol is mainly active against viruses with Cap-dependent translation initiation.

This finding is further substantiated by the effect of Silvestrol in another Picornavirus, namely Polioviruses, which also initiate translation via an IRES. Again, the effect of Silvestrol in Poliovirus infected VeroE6 cells is taking place with an EC₅₀ value of about 100 nM, a concentration where unwanted effects of Silvestrol against proto-oncogenes are expected.

### Antiviral activity of Silvestrol on EBOV-infected human primary macrophages

Primary human macrophages, which are one of the first target cells during EBOV infections, are a highly relevant cellular system to thoroughly investigate the antiviral effects of Silvestrol on EBOV replication. Using the above mentioned preincubation setup, the impact of Silvestrol on EBOV infection of macrophages isolated from two different donors at 2, 10 and 25 nM is analyzed. A concentration-dependent inhibition of viral titers between 1 to 3 log orders can be observed (Fig. 4A). Repetition of the experiment with macrophages isolated from more donors verifies the antiviral effect of Silvestrol: Preincubation of EBOV-infected macrophages from seven donors with 10 nM Silvestrol confirmes the reduction of viral titers by up to two orders of magnitude (Fig. 4B).

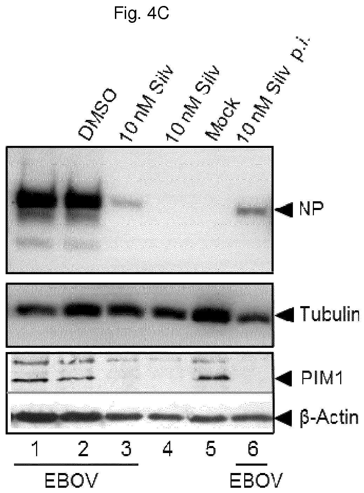

Upon analyzing the expression level of the EBOV NP protein by Westernblotting a potent downregulation is observed (Fig. 4C, example of one donor, lane 3). The same is true when Silvestrol is administered after EBOV infection (Fig. 4C, lane 6). As mentioned above, Silvestrol inhibits translation of highly structured 5'-UTRs of proto-oncogenes and thus leaves the expression of housekeeping genes largely unaffected. Therefore, the impact of Silvestrol on the levels of the proto-oncogenic kinase PIM1 in primary macrophages is tested, using β-Actin as a housekeeping control protein. As expected, PIM1 levels are strongly decreased (to almost below detection limits) in the presence of 10 nM Silvestrol (Fig. 4C).

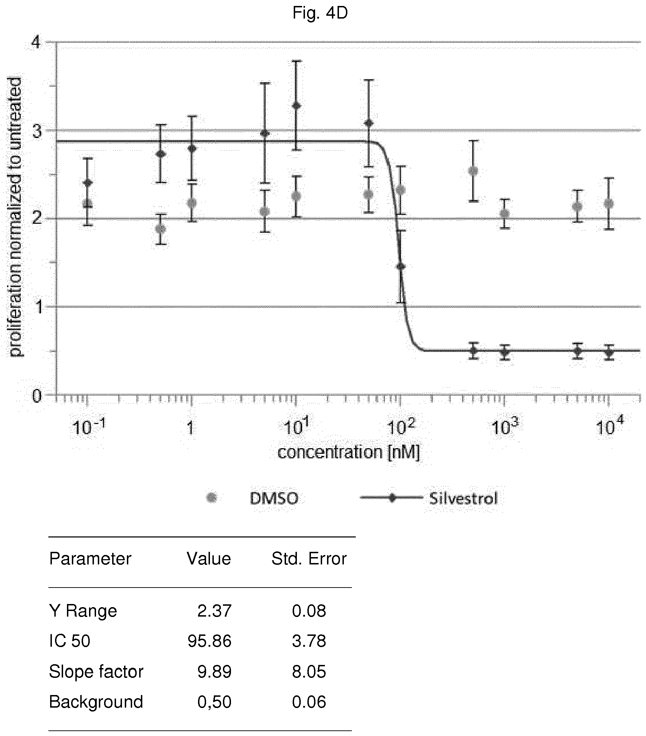

The toxicity of Silvestrol is quantified in primary macrophages using the WST-1 viability assay in a concentration range between 0.1 nM - 10 µM This reveales an IC₅₀ value of about 96 nM, with essentially no sign of toxicity up to at least 50 nM Silvestrol (Fig. 4D). In accordance with this result it is known in the state of the art that Silvestrol exerts no toxicity at therapeutic doses in antitumorigenic animal studies. The results revealed herein prove the existence of a therapeutic window from below 1 nM until more than 1 µM within which Silvestrol can be applied to efficiently inhibit EBOV propagation at non-toxic concentrations by affecting cap-dependent translation initiation of viral mRNAs.

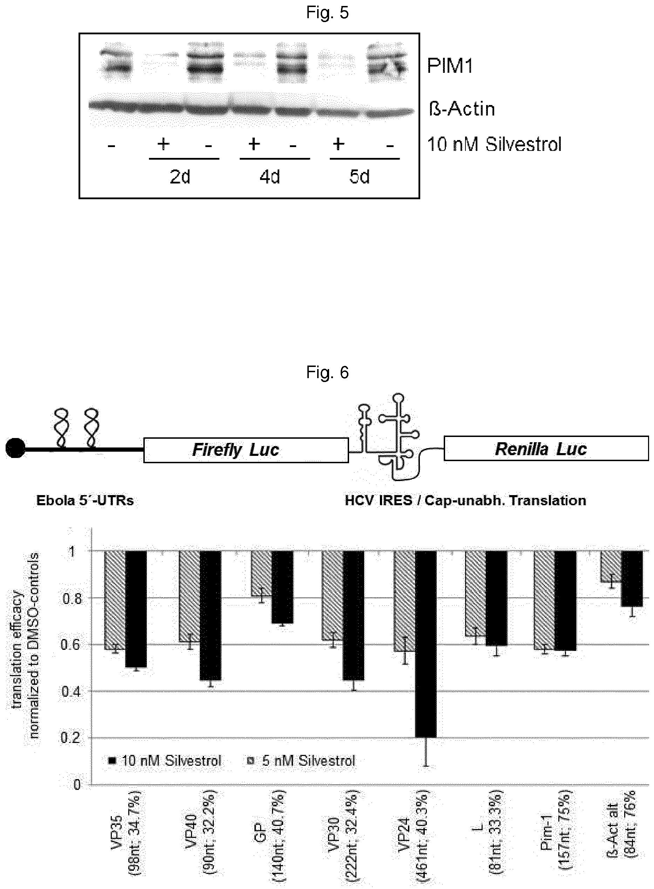

Importantly, this inhibitory effect (cf. Fig. 4C) continued for at least 5 days using a single dose of Silvestrol, without affecting cellular β-Actin levels (Fig. 5).

Fig. 6 shows results of experiments regarding the analysis of the 5'-UTRs of Ebola mRNAs by using a dual luciferase reporter gene assay. The 5'-UTRs of the EBOV genes VP35, VP40, GP, VP30, VP24 and L were cloned infront of the firefly luciferase gene to determine the effects of Silvestrol treatment on firefly luciferase expression. Additionally the reporter vector contain an IRES-driven renilla luciferase gene to quantify cap-independent translation for normalization of the plasmid transfection rate. Treating transfected cells with Silvestrol has no obvious effect on renilla luciferase activity. The effects of Silvestrol on firefly luciferase activity were concentration dependent with a maximum inhibitory effect of 80% in case of the plasmid that harbors the VP24 5'-UTR infront of the firefly luciferase reporter gene. The 5'-UTR of PIM1 was used as a positive control and the 5'-UTR of β-Actin as a negative control.

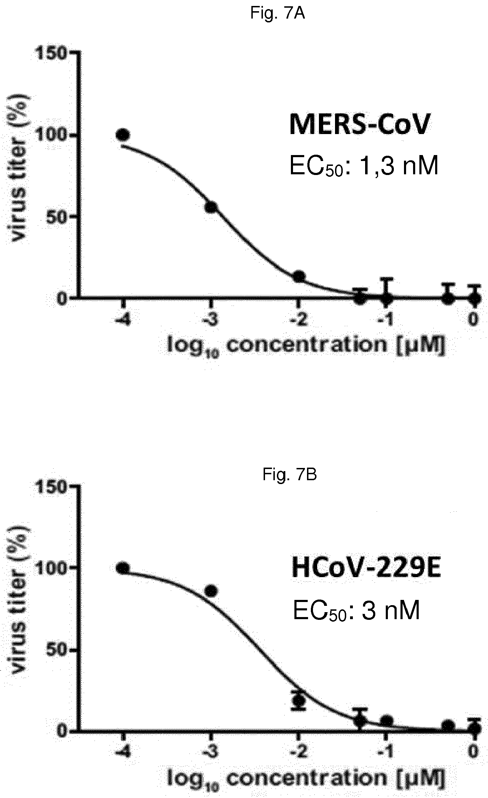

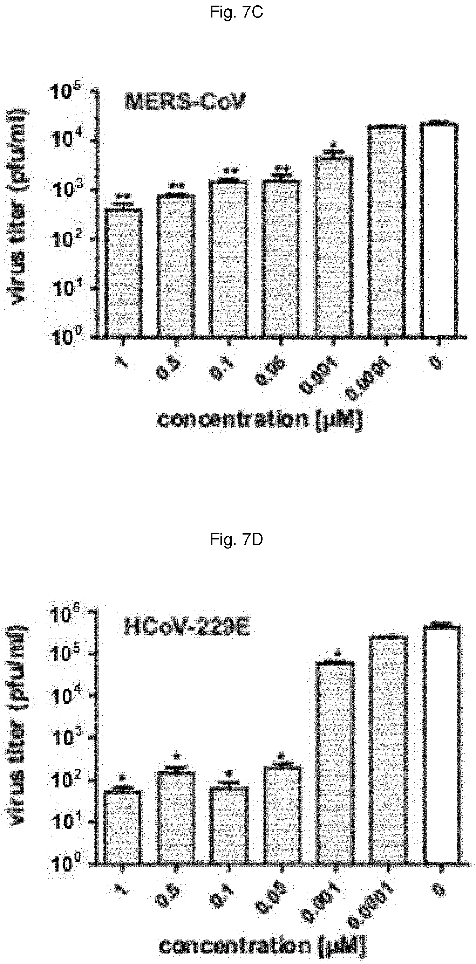

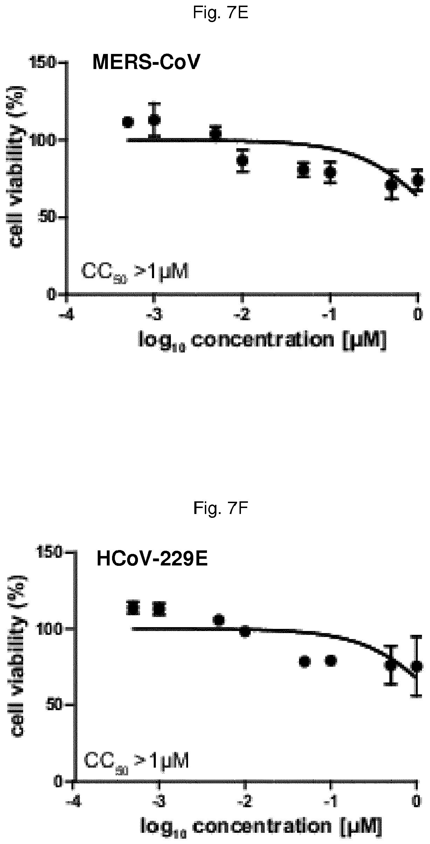

Silvestrol is also inhibiting the MERS-CoV (high pathogenic) and HCoV-229E (low pathogenic) replication in primary MRC-5 lung-cells, as can be seen on Fig. 7A/B/C/D. The viral titers of infected cells were determined 24 h post infection and EC₅₀-values were determined. The cell toxicity of Silvestrol in primary MRC-5 cells is shown in Fig. 7E/F (type MERS-CoV (highly pathogenic) and HCoV-229E (low pathogenic)) by analyzing the cell viability 24 h post infection. The data presented in Fig. 7A-F shows that:
- The cell toxicity of Silvestrol in MRC-5 cells is above 1 µM
- EC₅₀ of Silvestrol within cells of MERS-CoV type is about 1.3 nM
- EC₅₀ of Silvestrol within cells of HCoV-229E type is about 3 nM
- The selectivity index (CC₅₀:EC₅₀) is about 500 - 1000.

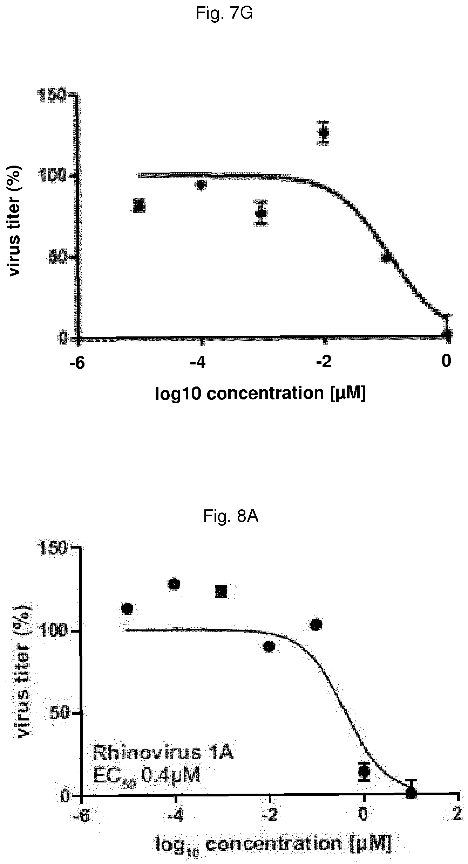

Regarding the IRES-dependent translation on cell line MRC5, infected with Rhinovirus 1 A, Fig. 7G shows that Silvestrol offers an EC₅₀-value of 0.1 µM and a CC₅₀-value of 1.7 µM Regarding the Poliovirus a CC₅₀-value of 2 µM is observed.

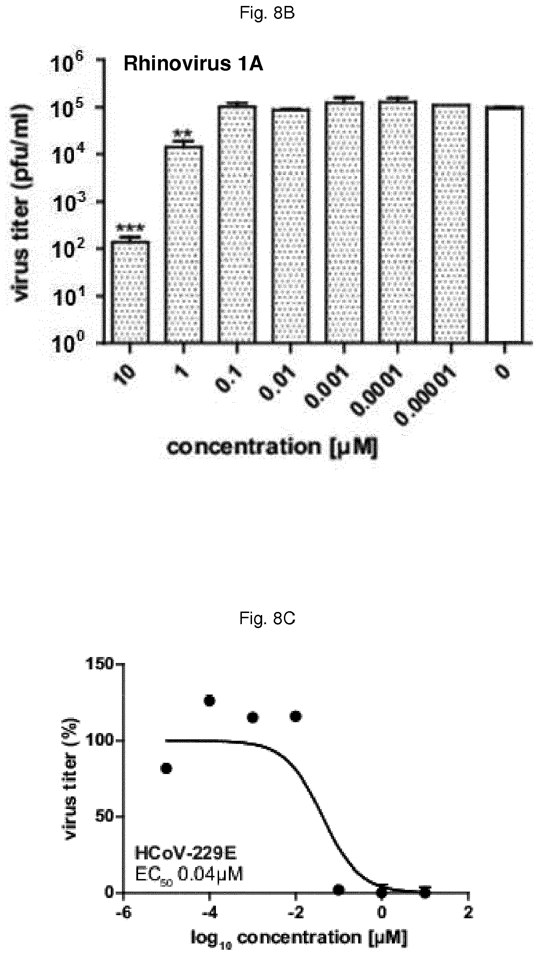

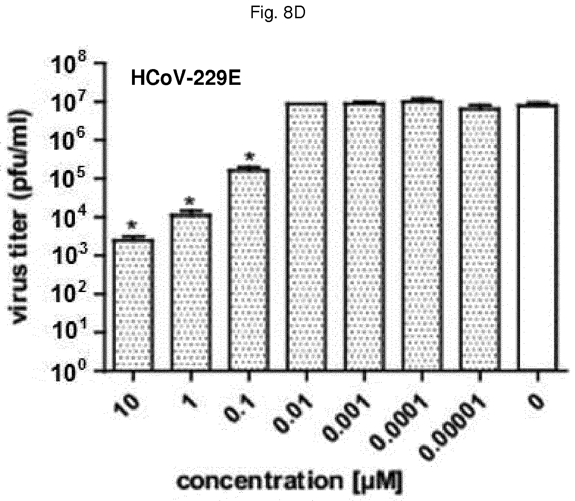

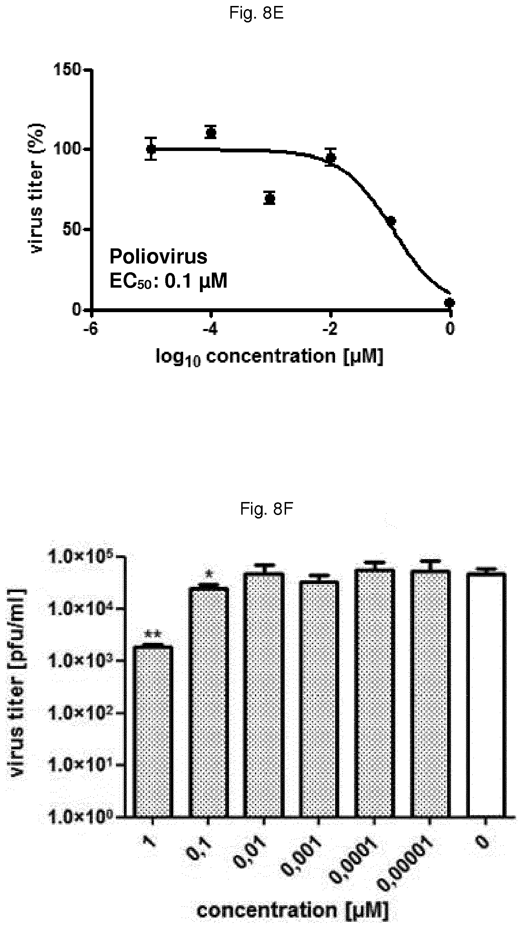

That there is no specific inhibition by Silvestrol of the translation of IRES-dependent viruses (Picornaviruses ((+) ssRNA), e.g. Rhinovirus 1A, Poliovirus) can be seen on Fig. 8A/B/C/D/E/F. The data presented in Fig. 8A-F shows that:
- With Rhinovirus 1 A (Picornavirus) the EC₅₀-value is about 400 nM.
- With Poliovirus (Picornavirus) the EC₅₀-value is about 100 nM.
- At concentrations of more than 50 nM Silvestrol within tumor-cells anti proliterative effects are generated via inhibition of proto-oncogenes.

Thus it is obvious to the person skilled in the art that the scope of the invention regarding disease control is not confined to the Ebola- and Marburgvirus, but comprises all types of RNA viruses which also perform cap-dependent protein translation, e.g. Coronaviridae, Flaviviridae like the Zikavirus and the Denguevirus, Togaviridae like the Chikungunyavirus, Bunyaviridae, Orthomyxo-, Rhabdo-, Paramyxoviridae etc.). This list is, of course, in no way limiting the scope of the invention.

Summarizing the invention, it may be described as follows:
The invention comprises a medicament comprising a therapeutic effective amount of Silvestrol and/or Episilvestrol and/or derivatives of Silvestrol or Episilvestrol and/or analoga of Silvestrol or Episilvestrol for the treatment of virus infections of humans and/or mammals.

To the person skilled in the art it is well known that the efficacy of an active ingredient of a medicament may often be improved, i.e. adapted regarding its efficacy, bioavailability, biodistribution, stability and/or compatibility, via chemical modification, e.g. etherification, esterification, sulfonation, substitution, elimination, cyclisation etc. For example, it is well known that fluorination, i.e. substitution of hydrogen atoms by fluorine atoms is prolonging the time span the active ingredient stays within the body of the patient, thus intensifying the efficacy. Therefore it is obvious to the person skilled in the art that any derivative of Silvestrol or Episilvestrol that shows better antiviral effects and/or less unwanted side effects lies within the scope of the invention. A sample list of possible substituents comprises -F, -Cl, -Br, -CH₃, -C₂H₅, -C₃H₇, -CF₃, -C₂F₅, -C₃F₇, -alkyl, -cycloalkyl, -aryl, -heteroaryl, -acyl, -SOₙR_{f}, -SOₙH, -POₘR_{f}, -POₘH, -SOzN(Rf)2; -POₘ(NR_{f})ₓ, -SO_{z}(NR_{f})_{y}, -B(OR_{f})₂, -OB(OR_{f})₂, -B(OR_{f})(NR_{f}), -B(NR_{f})₂, -OB(ORf)(NR_{f}), -OB(NR_{f})₂, substituted -aryl, substituted -heteroaryl, -alkylaryl, -alkylheteroaryl, -OH, -phenyl, -NH₂, -NO₂, -OR_{f}, -O-COR_{f}, -CORf, -N(R_{f})₂, -NR_{f}COR_{f}, -O-CSR_{f}, -CSR_{f}, -NR_{f}CSR_{f}, -O-CSeR_{f}, -CSeR_{f}, -NRfCSeR_{f}, -O-CN(R_{f})R_{f}, -CN(R_{f})R_{f}, -NR_{f}CN(R_{f})R_{f}, -N(R_{f})SO₂R_{f}, -N(R_{f})SO₂N(R_{f})₂, -SSR_{f}, -SR_{f}, -SOₓR_{f}, -SOₙN(R_{f})₂, -nitril, -amidine, -guanidine, -COOR_{f}, -CON(R_{f})₂, -CON(R_{f})N(R_{f})2, -COSR_{f}, -CSOR_{f}, -CSSR_{f}, -NR_{f}COR_{f}, -N(R_{f})N(R_{f})₂, -NHR_{f}, -NR_{f}R_{g}, -CONR_{f}R_{g}, -NHCOR_{f}, -NRfCOR_{g}, -CSRf, -CSNHRf, -CSNRfR_{g}, -CSR_{f}, -OCSR_{f}, -NR_{f}CSR_{f}, -NR_{f}CSR_{g}, -COSR_{f}, -CSNR_{f}N(R_{f})₂, -CONR_{f}N(R_{f})₂, -NR_{f}N(Rf)₂.

Substituents R_{f} and R_{g} are independently from each other chosen from the list comprising -H, -alkyl, -cycloalkyl, -aryl, -heteroaryl, substituted -aryl, substituted -heteroaryl, -alkylaryl, -alkylheteroaryl.

The substituents of the substituted -aryl and substituted -heteroaryl groups are chosen from the list comprising -F, -Cl, -Br, -OH, -phenyl, -NH₂, -NO₂, -OR_{f}, -O-COR_{f}, -COR_{f}, -N(R_{f})₂, -NR_{f}COR_{f}, -O-CSR_{f}, -CSR_{f}, -NR_{f}CSR_{f}, -O-CSeR_{f}, -CSeR_{f}, -NR_{f}CSeR_{f}, -O-CN(R_{f})R_{f}, -CN(R_{f})R_{f}, -NR_{f}CN(R_{f})R_{f}, -N(R_{f})SO₂R_{f}, -N(R_{f})SO₂N(R_{f})₂, -SSR_{f}, -SR_{f}, -SOₓR_{f}, -SOₙN(R_{f})₂, -nitrile, -amidine, -guanidine, -COOR_{f}, -CON(R_{f})₂, -CON(R_{f})N(R_{f})₂, -COSR_{f}, -CSOR_{f}, -CSSR_{f}, -NR_{f}COR_{f}, -N(R_{f})N(R_{f})₂ .

The formular indices "x" and "y" can adopt values from 1 to 2, formular index "n" can adopt values from 0 to 4 and formular index "m" can adopt values from 1 to 4.

This list is not limiting the scope of the invention, Silvestrol and Episilvestrol may be multiply substituted, the substituents are chosen independently from each other and may be attached to any part of the base molecule.

Mostly preferred derivatives of Silvestrol and/or Episilvestrol for a medicament according to the invention are derivatives according to the chemical formula **I**, 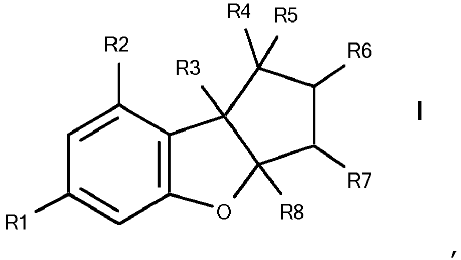 characterized in that R₁ and R₂ are independently chosen from the list comprising F, Cl, Br, Alkyl, Aryl, subst. Alkyl, subst. Aryl, Heteroaryl, subst. Heteroaryl, OH, OR₉, NR₁₀R₁₁, SH, SR₁₂, SOR₁₃, SOR₁₄, COOR₁₅, CONR₁₆R₁₇, SO₂NR₁₈R₁₉, CN and R₃ is independently chosen from the list comprising H, OH, OR₂₀, SR₂₁, NR₂₂R₂₃, F, Cl, Br, Alkyl, and R₄ and R₅ i) are either together representing a substituent chosen from the list comprising =O, =NR₂₄, =NOR₂₅, =CR₂₆R₂₇, =S or ii) R₄ is H and R₅ is independently chosen from the list comprising OH, NHR₂₄, NHOR₂₅, CHR₂₆R₂₇, SH, SR₂₈ and R₆ is independently chosen from the list comprising the substituents depicted by the chemical formula II through IX, 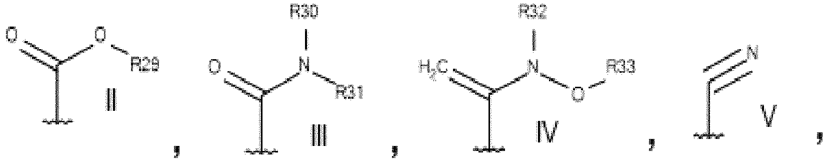 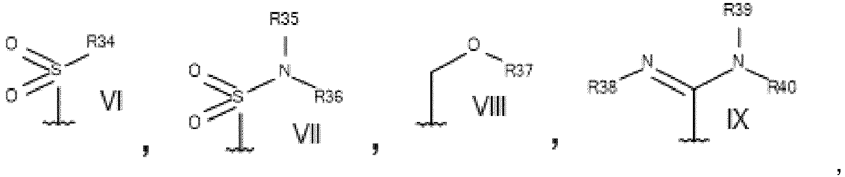 and R₇ and R₈ are independently chosen from the list comprising Aryl, subst. Aryl, Heteroaryl, subst. Heteroaryl, whereat R₉ through R₄₀ are independently chosen from the list comprising H, F, Cl, Br, OH, phenyl, NH₂, NO₂, alkyl, branced alkyl, cycloalkyl, aryl, hetero-aryl, substituted alkyl, substituted branched alkyl, substituted cycloalkyl, substituted aryl, substituted heteroaryl, alkylaryl, alkylheteroaryl, nitrile, amidine, guanidine.

Analogously it is well known to the person skilled in the art that so-called analoga of physiologically active substances exert the same biological effect, in this case antiviral activity, although they have chemically different structures. A very common example of this effect is the oestrogenic effect of numerous plasticisers, e.g. diethylphthalate, despite the fact that their chemical structure is very different from the structure of oestrogen. Therefore it is obvious to the person skilled in the art that any analogue of Silvestrol or Episilvestrol that shows similar antiviral effects and/or less unwanted side effects also lies within the scope of the invention. This is especially true if the antiviral effect of the analogue is based on the same mechanism (inhibition of cap-dependent translation).

The invention further comprises a medicament according to the previous paragraph, characterized in that the medicament has an antiviral effect against viruses with cap-dependent protein translation and has no specific antiviral effect against viruses with IRES-dependent protein translation, whereat "no specific antiviral effect against viruses with IRES-dependent protein translation" means that efficient inhibition of the virus titer of viruses with IRES-dependent protein translation occurs at concentrations higher than 500 nM, more preferred at concentrations higher than 100 nM and even more preferred at concentrations higher than 50 nM in infected cells. The virus titer considered here is the concentration of the virus within the body fluid relevant for the virus currently under consideration, determined via methods well known to the person skilled in the art. The expression "efficient inhibition" means within this context that the virus titer is reduced for about more than 10 per cent compared to the value without treatment with one or more of the inventive medicaments.

The invention further comprises analogously to the previous paragraph a medicament, characterized in that the efficient inhibition of the virus titer of viruses with IRES-dependent protein translation occurs at EC₅₀-values higher than 10000 nM, more preferred at EC₅₀-values higher than 1000 nM and even more preferred at EC₅₀-values higher than 100 nM in infected cells.

The invention further comprises a medicament according to one or more of the preceding paragraphs, characterized in that the medicament inhibits the propagation of ssRNA viruses with a (+) RNA genome or a (-) RNA genome.

The invention further comprises a medicament according to one or more of the preceding paragraphs, characterized in that the virus which is causing the virus infection is a virus with cap-dependent translation.

The invention further comprises a medicament according to one or more of the preceding paragraphs, characterized in that it is applied as a pharmaceutical acceptable composition that can be either inhaled, administered orally, rectally, intravenously, intramuscularly, subcutanously, mucocutaneously, intraperitoneally, intravascularly, transdermally, topically, buccally, intradermally, intragastrally, intracutaneously, intranasally, intrabuccally, inthrathecally, percutaneously, sublingually, or can be applied externally as powder, spray, ointment or as medical drops.This list of application techniques is just simply naming some of the most popular application techniques of medicaments exemplarily and thus is not limiting the scope of the invention. It is obvious to the person skilled in the art that other application methods that allow the effective application of the inventive medicament are also included within the scope of the invention. Thus the list of pharmaceutical forms according to the invention comprises drops, oral sprays, nasal sprays, tablets, film-tablets, layer-tablets, suppositories, gels, ointments, syrups, inhalation powders, granulates, emulsions, dispersions, microcapsules, capsules, powder, injection-solutions, infusion-solutions, encapsulations within vesicles as are well known within dermatological/pharmaceutical industry for transporting compounds into and/or through the skin, e.g. anionic or cationic liposomes, niosomes, nano particles, multilamellar vesicles. The multilamellar vesicles may also be used for penetrating cells of skin or hair.

The medicament according to the invention may be produced and/or applied as any of the aforementioned pharmaceutical forms whereat this list is not to be understood as being confining the invention to the mentioned pharmaceutical forms. It is well known to the person skilled in the art that any pharmaceutical form providing sufficient stability to Silvestrol and/or Episilvestrol and/or derivatives of Silvestrol or Episilvestrol and/or analoga of Silvestrol or Episilvestrol as well as providing suitable kinetics of releasing of Silvestrol and/or Episilvestrol and/or derivatives of Silvestrol or Episilvestrol and/or analoga of Silvestrol or Episilvestrol lies within the scope of the invention.

The invention further comprises a medicament according to one or more of the preceding paragraphs, characterized in that it contains supplementary substances, chosen from the list comprising Citric Acid, Sodium Benzoate, Polyacrylic Acid, Calcium Carbonate, Starch, Lactose Sorbitol, Sucrose, Cellulose, Magnesiumstearate, Dicalciumphosphate, Calciumsulfate, Talc, Mannitol, Ethyl-alcohol, Methylcellulose, Polyvinyl Alcohol, denaturated Gelatine, Sodium-Carboxymethylstarch, natural and/or synthetic Gum like e.g. Arabic Gum, Carob Gum, Karaya Gum, Guar Gum, Tragacanth Gum, Agar, Cellulose derivatives like Methylcellulose, Sodium-Carboxymethylcellulose, microcrystalline Cellulose, Alginate, Alumina, Bentonite, Sugar, Starch from Grain, Rice or Potato, natural Gum like Acacia Gum, Gelatin, Traganth, Alginic Acid, Sodium Alginate, Ammonium-Calcium-Alginate, Methylcellulose, Cera, Sodium-Carboxymethylcellulose, Hydroxypropylmethylcellulose, Polyethylenglycole, Polyvinylpyrrolidone, inorganic compounds like Magnesium-Aluminum-Silicates, Boracic Acid, Stearates e.g. Magnesiumstearat, Calciumstearat, Potassiumstearat, Stearic Acid, high-melting Cera, Sodium-Chloride, Sodium-Benzoate, Sodium-Acetate, Sodium-Oleate, Polyethylenglycol, Amino-Acids like Leucine, Triglycerides of saturated Fatty Acids from plants, e.g. Miglyol 812^{®}, Triglycerin-Diisostearate, Esters of long-chain acids, e.g. Oleyl-Oleate, Isopropylmyristate, Isopropylpalmitate, 2-Ethylhexylpalmitate, Isooctylstearate, Isopropylstearate, Lauric Acid-Hexylester, Di-n-butyladipate, long-chain alkoholes, e.g. Hexylalcohol, Octylalkohol, Decylalkohol, Oleylalkohol, 2-Octyldodecanol, 2-Hexyldecanol, 2-Octyldecanol.

This list of supplementary substances is just simply naming some of the most popular supplementary substances exemplarily and thus is not limiting the scope of the invention. It is obvious to the person skilled in the art that other supplementary substances that support the storability or applicability of the inventive medicament are also included within the scope of the invention. Additionally the inventive medicament may contain colouring, flavouring, and/or binding agents. Liquid formulations comprise solutions, suspensions, dispersions, sprays and/or emulsions, e.g. aqueous solutions for injections or injection-solutions based on water and Propylenglycole, applicable for parenteral injections.

The aforementioned supplementary substances serve different purposes and are added in varying amounts, e.g.:
- as pharmacologically compatible carrier-substances, amounting from 5 to 95 per cent by weight of the inventive medicament;
- as substance supporting the dissolving of the medicament after application ("blasting means"), amounting from 2 to 30 per cent by weight of the inventive medicament;
- as binding agents, amounting from 1 to 30 per cent by weight of the inventive medicament;
- as lubricant, amounting from 1 to 20 per cent by weight of the inventive medicament;
- as penetrating agent, amounting from 5 to 95 per cent by weight of the inventive medicament

Any supplementary substance may be applied either alone or in combination with at least one other supplementary substance; especially the penetrating agents may be applied either alone or in combination with other penetrating substances. An especially advantageous effect regarding the penetration of medicaments offer long-chain alcohols, mostly 2-alcylic-substituted alcohols. The oils are applied at concentrations ranging from 5 to 95 per cent by weight, more preferred at concentrations ranging from 30 to 90 per cent by weight. The active components (Silvestrol and/or Episilvestrol and/or derivatives of Silvestrol or Episilvestrol and/or analoga of Silvestrol or Episilvestrol) can also be dispersed in water containing emulgated penetration supporting oils, whereat the water-content of these dispersions may be up to 50 per cent by weight, preferredly up to 30 per cent by weight.

The way of dosing of the inventive medicament is determined by the medicating person according to the clinical factors. It is well known to the person skilled in the art that the way of dosing is dependent from numerous criteria, e.g. body size, body weight, body surface, age, gender, the general health situation of the patient, the medicament to be administered, the duration and the way of administering the medicament and also other medicaments which may be administered at the same time, shortly before or afterwards.

The inventive medicament may be combined with at least one other agent, e.g. analgetics and/or antipyretics. Furthermore, it is possible to combine the inventive medicament with at least one anti-inflammatory medicament, immuno-modulator, anti-asthmatic, and/or broncho-dilatator. Also it is possible to combine the inventive medicament with at least one antibacterial, antifungal, anthelmintic and/or antiviral agent. Alternatively or additionally it is possible to combine the inventive medicament with at least one dermatologic and/or cosmetic agent.

The inventive medicament will be - according to the way of its manufacturing/production and its application-technology - used together with liquid and/or solid adjuvants, which are well known to the person skilled in the art, in order to acquire dilutable solutions, emulsions, dissolvable powders, granules, microencapsulations in polymeric substances or nano particles. It is - according to the way of administering - manufactured/produced according to a method well known to the person skilled in the art, e.g. by way of thorough mixing and/or grinding of the agents/substances/compounds and/or by addition of solvents and/or solid carrier-substances.

The invention further comprises a medicament according to one or more of the preceding paragraphs, characterized in that it contains the effective amount of Silvestrol and/or Episilvestrol and/or derivatives of Silvestrol or Episilvestrol and/or analoga of Silvestrol or Episilvestrol in protonated or deprotonated form, i.e. as salt.

The medicament containing Silvestrol and/or Episilvestrol and/or derivatives and/or analogs of Silvestrol and/or Episilvestrol is advantageously applied after an infection has taken place, but may also be applied in a preventive manner for the treatment of humans and/or mammals.

### Description of the drawings

- Fig. 1:: Scheme, showing the formation of the translation initiation complex eIF4F that includes the eIF4A helicase activity allowing the bonding of the 43S preinitiation complex; initiator: Met-charged initiator tRNA.
- Fig. 2A:: Toxicity profile of Silvestrol in the hepatoma cell line Huh-7: Proliferation of Huh-7 cells measured by a WST-1 assay. No effect of Silvestrol on proliferation could be observed up to a concentration of 10 nM. At higher concentrations up to 100 nM Silvestrol, weak effects on proliferation compared to the DMSO control could be observed.
- Fig. 2B:: Antiviral effects of Silvestrol in EBOV-infected Huh-7 cells: Addition of Silvestrol to a final concentration of 10 nM 2 h before viral infection strongly inhibits EBOV replication in Huh-7 cells. Samples from supernatants are collected at days 1, 2, and 3 post infection (p.i.) and subjected to TCID₅₀ analysis in VeroE6 cells. Viral titers determined for EBOV-infected cell cultures in the absence of Silvestrol or DMSO were set to 100%. Statistical significance of the results are indicated by p-values: *: p-value ≤0.05; ***: p-value ≤ 0.001.
- Fig. 2C:: Antiviral effects of Silvestrol in EBOV-infected Huh-7 cells: Morphology of Huh-7 cells in the light microscope after three days of Silvestrol treatment (10 nM) in the presence or absence of EBOV. Pictures were taken at 10x magnification.
- Fig. 2D:: Antiviral effects of Silvestrol in EBOV-infected Huh-7 cells: Silvestrol inhibits the expression of the EBOV protein VP40, whereas the cellular protein tubulin is not affected. EBOV-infected Huh-7 cells were treated with DMSO or 10 nM Silvestrol 2 h before (lane 2) or immediately post (lane 3) infection. Shown are the levels of the viral protein VP40 and the cellular protein tubulin as control in a Westernblot analysis from samples obtained at 3 days p.i. Mock: uninfected cells without Silvestrol or DMSO addition.
- Fig. 2E:: Downregulation of the Ebola virus proteins GP and NP by Silvestrol in EBOV-infected Huh-7 cells with 10 or 50 nM Silvestrol.
- Fig. 3:: Dose-dependent inhibition of Ebola virus propagation by Silvestrol in Huh-7 cells. Cells are treated with DMSO as a control or with 1, 5 or 10 nM Silvestrol 2 h before EBOV-infection or with 10 nM Silvestrol immediately post EBOV-infection.
- Fig. 4A:: Antiviral effects of Silvestrol in EBOV-infected primary human macrophages: Macrophages from two human donors were infected with EBOV and cell culture supernatants were subjected to TCID₅₀ analysis 3 days post infection. Addition of Silvestrol to 2, 10 or 25 nM 2 h before infection resulted in a concentration-dependent inhibition of EBOV propagation by Silvestrol between 1 to 3 orders of magnitude compared to DMSO-treated cells.
- Fig. 4B:: Antiviral effects of Silvestrol in EBOV-infected primary human macrophages: Similar results (cf. Fig. 4A) are obtained with an enlarged set of EBOV-infected macrophages from seven human donors upon addition of Silvestrol to 10 nM 2 h before infection.
- Fig. 4C:: Antiviral effects of Silvestrol in EBOV-infected primary human macrophages: Westernblot analysis demonstrating strongly decreased levels of the EBOV protein NP and the proto-oncoprotein PIM1 upon treatment of macrophages at 10 nM Silvestrol. Tubulin and β-Actin are shown as cellular control proteins.
- Fig. 4D:: Antiviral effects of Silvestrol in EBOV-infected primary human macrophages: Silvestrol inhibited proliferation of human macrophages with an IC₅₀ value of 96 nM, as determined by a WST-1 viability assay.
- Fig. 5:: Long-term effect of Silvestrol analyzed by Westernblotting. The inhibitory effect of a single-dose treatment with 10 nM Silvestrol on PIM1 levels persists in macrophages up to at least 5 days without affecting β-Actin levels.
- Fig. 6:: Dual Luciferase-assay for analysis of the 5'-UTRs of Ebola mRNAs showing a dose-dependent inhibitory effect of 5 or 10 nM Silvestrol on reporter gene expression. The inhibitory effects of 10 nM Silvestrol on luciferase levels were between 30% for GP and 80% for VP24. Silvestrol has no effect on the IRES-driven expression of the renilla luciferase gene thus allowing normalization of the firefly luciferase levels by renilla luciferase activity.
- Fig. 7A:: Effect of Silvestrol in the human lung fibroblast-like cell line MRC5 infected with MERS-CoV (MOI of 0.1). Virus titers are determined 24 hours post transfection. An effective dose (EC₅₀) of 1.3 nM for Silvestrol is determined as well as a CC₅₀-value of 2 µM.
- Fig. 7B:: Effect of Silvestrol in the human lung fibroblast-like cell line MRC5 infected with HCoV-229E (MOI of 0.1). Virus titers are determined 24 hours post transfection. An effective dose (EC₅₀) of 3 nM for Silvestrol is determined as well as a CC₅₀-value of 1.7 µM
- Fig. 7C:: Effect of Silvestrol in the human lung fibroblast-like cell line MRC5 infected with MERS-CoV (MOI of 0.1). Virus titers are determined 24 hours post transfection. Bar diagrams showing the inhibitory effect of Silvestrol (virus titers are determined as plaque forming units per ml) in a concentration range between 1 µM and 0.1 nM.
- Fig. 7D:: Effect of Silvestrol in the human lung fibroblast-like cell line MRC5 infected with HCoV-229E (MOI of 0.1). Virus titers are determined 24 hours post transfection. Bar diagrams showing the inhibitory effect of Silvestrol (virus titers are determined as plaque forming units per ml) in a concentration range between 1 µM and 0,1 nM.
- Fig. 7E:: Determination of cell cytotoxicity of Silvestrol in MRC5 cells. CC₅₀ values are about 2 µM for Silvestrol at 37°C (conditions for MERS-CoV infection).
- Fig. 7F:: Determination of cell cytotoxicity of Silvestrol in MRC5 cells. CC₅₀ values are about 1.7 µM at 33°C (conditions for HCoV-229E infection).
- Fig. 7G:: Effect of Silvestrol upon IRES-dependent translation on cell line MRC5, infected with Rhinovirus 1A (24h post infection), showing an EC₅₀-value of 0.1 µM and a CC₅₀-value of 1.7 µM
- Fig. 8A:: Effect of Silvestrol in HeLa cells infected with Rhinovirus 1A (MOI of 0.1). Virus titers are determined 24 hours post transfection. An effective dose (EC₅₀) of 0.4 µM for Silvestrol is determined.
- Fig. 8B:: Effect of Silvestrol in HeLa cells infected with Rhinovirus 1A (MOI of 0.1). Virus titers are determined 24 hours post transfection. Bar diagrams showing the inhibitory effect of Silvestrol (virus titers are determined as plaque forming units per ml) in a concentration range between 10 µM and 0.01 nM.
- Fig. 8C:: Effect of Silvestrol in Huh-7 cells infected with HCoV-229E (MOI of 0.1). Virus titers are determined 24 hours post transfection. An effective dose (EC₅₀) of 0.04 µM for Silvestrol is determined.
- Fig. 8D:: Effect of Silvestrol in Huh-7 cells infected with HCoV-229E (MOI of 0.1). Virus titers are determined 24 hours post transfection. Bar diagrams showing the inhibitory effect of Silvestrol (virus titers are determined as plaque forming units per ml) in a concentration range between 10 µM and 0.01 nM.
- Fig. 8E:: Effect of Silvestrol in VeroE6 cells infected with Poliovirus (MOI of 0.1). Virus titers are determined 24 hours post transfection. An effective dose (EC₅₀) of 0.1 µM for Silvestrol is determined.
- Fig. 8F:: Effect of Silvestrol in VeroE6 cells infected with Poliovirus (MOI of 0.1). Virus titers are determined 24 hours post transfection. Bar diagrams showing the inhibitory effect of Silvestrol (virus titers are determined as plaque forming units per ml) in a concentration range between 1 µM and 0.01 nM.

### Detailed embodiments of the invention

### Cells and viruses

Human hepatocyte derived cellular carcinoma cells (Huh7), African Green monkey kidney cells (VeroE6), human cervical cancer cells (Hela) and primary lung fibroblasts (MRC-5) are grown in Dulbecco modified Eagle medium (DMEM) supplemented with 10 % FCS, 100 U/ml penicillin and 100 µg/ml streptomycin at 37° C and 5 % CO₂.

Human Coronavirus 229E (HCoV-229E), MERS-CoV (EMC/2012), human Rhinovirus 1 A (HRV1 A) and Poliovirus Typ 1 (strain Mahoney; PV) are provided by the virus strain collection of the Institute of Medical Virology, Giessen, Germany.

### Ebola virus infection

All work with Zaire Ebola virus (EBOV) strain Mayinga (Acession number AF 086833) is performed at the biosafety level 4 (BSL4) laboratory of the Philipps University Marburg. Huh-7 cells (5 x 10⁵ cells) or primary human macrophages (3 x 10⁶ cells) are infected with EBOV at a multiplicity of infection (MOI) of 0.1 for 1 h at 37°C. Then Inoculum is removed and cells are incubated in Dulbecco's modified Eagle medium (DMEM) containing 3% fetal calf serum supplemented with Silvestrol or DMSO as indicated.

Aliquots of the supernatant are collected at 1-4 days post infection (p.i.) and subjected to the 50% tissue culture infectious dose TCID₅₀ assay for determination of viral titers. Cells are lysed with 1% SDS, followed by Westernblotting to analyze for expression of viral proteins and the cellular PIM1 kinase.

### Silvestrol treatment of EBOV-infected cells

Silvestrol is added to cell cultures as a single dose to final concentrations of 1 to 25 nM (dissolved from a 6 mM stock in DMSO, dilutions in DMEM). Silvestrol is added (Huh-7: one day post seeding; primary human macrophages: 7 days post seeding) 2 h before infection or directly post infection. As a control, DMSO is added to cells at the same concentration used to dissolve Silvestrol. Infected cells are incubated for 1 to 4 days at 37°C in growth medium containing the indicated Silvestrol concentrations.

### TCID₅₀ analysis

Virus titers in the supernatant of infected cells are determined by the 50% tissue culture infectious dose (TCID₅₀) assay in VeroE6 cells as is well known to the person skilled in the art.

### Cell culture and preparation of macrophages from PBMCs

Huh-7 (human hepatoma) and VeroE6 (African green monkey, kidney epithelia) cells are cultivated in Dulbecco's modified Eagle medium complemented with penicillin (100 U/ml), streptomycin (100 mg/ml), 5 mM L-glutamine and 10% fetal calf serum at 37°C and 5% CO₂ in a humidified atmosphere.

Human PBMCs (Peripheral Blood Mononuclear Cells) are isolated from buffy coats by density gradient centrifugation and magnetic CD14 MicroBeads according to the manufacturer's protocol (Miltenyi Biotec, Bergisch Gladbach, Germany). Monocytes (3 x 10⁶ cells) are cultivated in 6-well tissue culture plates (Falcon Primaria, Becton Dickinson, Paramus, NJ) at 37°C in 5% CO₂ using RPMI 1640 medium (GE Healthcare, Freiburg, Germany) supplemented with 2 mM L-Glutamine, 1 mM sodium pyruvate, 100 U/ml penicillin, 100 µg/ml streptomycin and 1% non-essential amino acids. After 2 h, defibrinated human AB serum is added to a final concentration of 2%, and GM-CSF (Biochrom, Berlin, Germany) to a final concentration of 10 ng/ml. Cells are cultivated for 7 days to differentiate them into macrophages. On day 3 post seeding half of the medium is replaced with fresh medium (including all supplements). Alternatively, preparation of monocytes from PBMCs is performed by counterflow centrifugation.

### WST-1 assay of human Huh-7 cells or primary macrophages

Huh-7 cells (7 x 10³ cells in 200 µl IMDM, 10% FCS) are seeded in 96-well microplates and incubated for 24 h (37°C, 5% CO₂). The medium is replaced with fresh medium, and Silvestrol or DMSO is added as indicated for 48 h. The medium is aspirated and 110 µL of 10% WST-1 reagent (Roche, Mannheim, Germany) in PBS is added. Absorbance is measured using a Tecan Safire II (measurement wavelength: 450 nm; reference wavelength: 600 nm).

Monocytes (8 x 10⁴ cells in 100 µL RPMI 1640) are seeded in 96-well microplates and incubated for 2 h (37°C, 5% CO₂). Then 100 µL RPMI 1640 supplemented with defibrinated human AB serum (final concentration 2%) and GM-CSF (final concentration 10 ng/mL) (Biochrom, Berlin, Germany) is added. After 72 h, half of the medium is removed and replaced with RPMI 1640 containing 4% human serum. After 96 h, the monocytes are differentiated into macrophages. The medium is then replaced with RPMI 1640 containing 2% human serum, and Silvestrol or DMSO is added as indicated followed by incubation for another 72 h. The WST-1 assay is then performed as described above for Huh-7 cells.

### Westernblot analysis

Cells are lysed in lysis buffer (125 mM Tris/HCl pH 6.8, 4% SDS, 1.4 M 2-mercaptoethanol, 0.05% bromophenol blue) and heated at 95 °C for 5 min. Samples are loaded onto 15% SDS-polyacrylamide gels followed by electrophoresis for 1 h at 180 V. Proteins are transferred onto an Immobilon-P PVDF membrane (Merck Millipore, Darmstadt, Germany) for 30 min at 10 V followed by blocking of the membrane with 5% milk powder dissolved in TBST (10 mM Tris/HCl, 150 mM NaCl, 0,1% Tween 20, pH 7.6). Primary and secondary antibodies are diluted 1:500 in TBST (Pim1, sc-13513, Santa Cruz Biotechnology), 1:5000 (β-Actin, sc-47778, Santa Cruz Biotechnology) and 1:5000 (goat anti-mouse IgG-HRP, secondary antibody). Blots are incubated with Amersham ECL™ or ECLplus™ Western Blotting Detection Reagents according to the manufacturer's protocol. For detection of chemiluminescence, Kodak^{®} BioMaxTM light films, Kodak GBX Developer and Replenisher and GBX Fixer and Replenisher are used.

Samples from infected cells are lysed in 1% SDS containing sample buffer and subjected to 12% SDS PAGE and semi-dry transfer onto nitrocellulose membranes. Staining of viral proteins from infected Huh-7 cells is performed using a goat anti-VP40 antibody and a donkey anti-goat Alexa680nm antibody (Thermo Fisher Scientific/Molecular Probes). Detection of antibodies is performed using the Odyssey Infrared Imaging System (LI-COR, Lincoln, NE, USA). Staining of viral proteins from human primary macrophages is performed using a chicken anti-NP antibody (at a 1:1000 dilution) and a donkey anti-chicken peroxidase-coupled antibody (Dianova). Tubulin is stained using mouse anti-tubulin (SIGMA) and goat anti-mouse peroxidase-coupled antibody (DAKO). Detection of antibodies is performed using the ChemiDoc™ XRS+ System (BIO-RAD).

### Antiviral activity of Silvestrol against Coronaviruses

MRC-5 cells are infected with the low pathogenic HCoV-229E or with the high pathogenic MERS-CoV at a MOI of 0.1. The cell cytotoxicity of Silvestrol in MRC-5 cells is analyzed at 33°C (temperature optimum for HCoV-229E) and 37°C (temperature optimum for MERS-CoV) and the cell cytotoxicity is measured by determining CC₅₀values of about more than 1 µM under both conditions. Importantely, low doses of Silvestrol efficiently reduce the viral titers resulting in an EC₅₀ value of 1.3 nM for MERS-CoV infected- and an EC₅₀ value 3 nM for HCoV-229E infected MRC-5 cells. Thus, a selectivity index (CC₅₀/EC₅₀) for Silvestrol of about 500 is observed.

### Dual-Luciferase Reporter Assay (cf. Fig. 6)

The day before transfection 2x10⁴ HepG2 cells per well are seeded in a 98-well plate (Greiner Bio One International GmbH, cat. #655090) in 200 µL IMDM (Lonza, cat. #12-722F) supplemented with 10% FCS (Biochrom AG, cat. #S0115) and incubated at 37°C, 5% CO2. The transfection is done in 100 µL Opti-MEM (Thermo Fisher Scientific Inc., cat. #51985026). 0.5 µg of the reporter construct DNA is diluted in 50 µL of Opti-MEM. 0.5 µL of Lipofectamine® 2000 Transfection Reagent (Thermo Fisher Scientific Inc., cat. #11668019) is used per transfection and therefore diluted in 50 µL of Opti-MEM. After 5min of incubation at room temperature both components are mixed and incubated for another 15 min at room temperature. Then complexes are added to the Opti-MEM media in the wells and incubated at 37°C, 5% CO₂. 4-6 h later the medium is aspirated and substituted with fresh medium (IMDM + 10 % FCS) containing the determined concentration of Silvestrol in sterile filtrated DMSO (Carl Roth GmbH + Co. KG, cat. #4720.4) respectively DMSO only. The cells are incubated for 48 h at 37°C, 5% CO₂ before performing the Dual-Luciferase^{®} Reporter Assay System (Promega Corporation, cat. #1960) according to the manual instructions. Measurements are done in the Tecan Safire 2 Multimode Reader.

### Antiviral Assays

To analyse the antiviral activity of Silvestrol, confluent monolayers (48 well format, 1,9x10⁴ cells per well) of the respective cell lines are infected with HCoV-229E, MERS-CoV (on Huh7- and MRC-5 cells), HRV1A (on Hela- and MRC-5 cells) or PV (on Vero- and MRC-5 cells) with an MOI of 0.1. For HCoV-229E and HRV1A the cells are incubated at 33°C, whereas MERS-CoV and PV are incubated at 37°C. After 1 h of absorption the virus inoculum is removed, cells are rinsed with PBS and fresh medium without FCS containing the indicated concentrations of Silvestrol or corresponding DMSO solvent is added. 24 h p.i. supernatant was collected and stored at -80°C till assayed.

### Plaque Assay

Virus titers are determined via plaque assay. Therefore Hela cells (for RV), Vero cells (for PV), Huh7 cells (for HCoV-229E and MERS-CoV) are seeded in 6-well plates (cells should be 90-100% confluent, ∼9,5x10⁵) and inoculated with 10-fold virus dilutions in PBS++/BA/P/S (PBS containing 0.2 % BSA, 1 mM MgCl₂, 0.9 mM CaCl₂, 100 U/ml penicillin and 100 mg/ml streptomycin) for 1 h. Afterwards Avicel-containing medium (1x MEM, 1.25 % Avicel, FMC Biopolymer) is added to the cells. After 2-4 d incubation (depending on virus type) the plates are washed with PBS, fixed with 3.7 % PFA in PBS and the cell layer is stained with 0.15 % Crystal violet in PBS. Effective concentration 50 (EC50) is calculated using GraphPad Prism 5.0.

### Cell toxicity

The toxicity of Silvestrol is evaluated by incubation of the respective cells (cells should be 90-100% confluent, ∼1x10⁴ cells per well) with serial dilution of the compound in DMEM supplemented with 100 U/ml penicillin and 100 µg/ml streptomycin for 24 h in 96 well formats. Afterwards the medium is exchanged by 200 µl of MTT-mix (DMEM supplemented with 10 % FCS containing 175 µg/ml tetrazolium bromide, Sigma), which is added to each well. Cells are then further incubated for 90-120 Min. at 37°C and subsequently fixed in 3.7 % PFA (in PBS) for 30 min. Fixing solution is aspirated and the cells are air dried. Tetrazolium crystals are dissolved by adding 200 µl isopropanol to each well and photometrically analyzed at 490 nm excitation by ELISA reader (BioTek). Cytotoxic concentration 50 (CC₅₀) is calculated using GraphPad Prism 5.0.

## Claims

1. A medicament comprising a therapeutic effective amount of Silvestrol and/or Episilvestrol and/or derivatives of Silvestrol or Episilvestrol and/or analoga of Silvestrol or Episilvestrol for the treatment of virus infections of humans and/or mammals.

2. A medicament according to claim 1, **characterized in that** the medicament has an antiviral effect against viruses with cap-dependent protein translation and has no specific antiviral effect against viruses with IRES-dependent protein translation, whereat efficient inhibition of the virus titer of viruses with IRES-dependent protein translation occurs at concentrations higher than 500 nM, more preferred at concentrations higher than 100 nM and even more preferred at concentrations higher than 50 nM in infected cells.

3. A medicament according to claim 1, **characterized in that** the efficient inhibition of the virus titer of viruses with IRES-dependent protein translation occurs at EC₅₀-values higher than 10000 nM, more preferred at EC₅₀-values higher than 1000 nM and even more preferred at EC₅₀-values higher than 100 nM in infected cells.

4. A medicament according to one or more of the preceding claims, **characterized in that** the medicament inhibits the propagation of ssRNA viruses with a (+) RNA genome or a (-) RNA genome.

5. A medicament according to one or more of the preceding claims, **characterized in that** the virus which is causing the virus infection is a virus with cap-dependent translation.

6. A medicament according to one or more of the preceding claims, **characterized in that** the virus which is causing the virus infection is a virus contained in the list comprising the Ebola virus, the Marburg virus, Coronaviruses, the Zikavirus, the Denguevirus, Togaviridae like the Chikungunyavirus, Bunya-, Orthomyxo-, Rhabdo- and Paramyxoviridae.

7. A medicament according to one or more of the preceding claims, **characterized in that** it is applied as a pharmaceutical acceptable composition that can be either inhaled, administered orally, rectally, intravenously, intramuscularly, subcutanously, intraperitoneally, intravascularly, or can be applied externally as powder, spray, ointment or as medical drops.

8. A medicament according to one or more of the preceding claims, **characterized in that** it comprises at least one derivative of Silvestrol and/or Episilvestrol according to the chemical formula **I**, 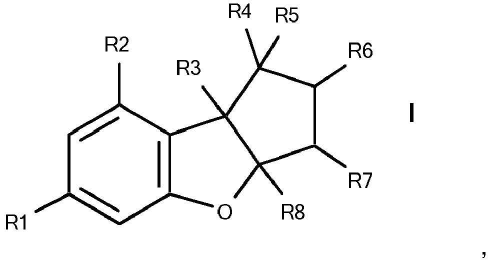 **characterized in that**
- R₁ and R₂ are independently chosen from the list comprising F, Cl, Br, Alkyl, Aryl, subst. Alkyl, subst. Aryl, Heteroaryl, subst. Heteroaryl, OH, OR₉, NR₁₀R₁₁, SH, SR₁₂, SOR₁₃, SOR₁₄, COOR₁₅, CONR₁₆R₁₇, SO₂NR₁₈R₁₉, CN and
- R₃ is independently chosen from the list comprising H, OH, OR₂₀, SR₂₁, NR₂₂R₂₃, F, Cl, Br, Alkyl and
- R₄ and R₅
i) are either together representing a substituent chosen from the list comprising =O, =NR₂₄, =NOR₂₅, =CR₂₆R₂₇, =S or
ii) R₄ is H and R₅ is independently chosen from the list comprising OH, NHR₂₄, NHOR₂₅, CHR₂₆R₂₇, SH, SR₂₈ and
- R₆ is independently chosen from the list comprising the substituents depicted by the chemical formula II through IX, 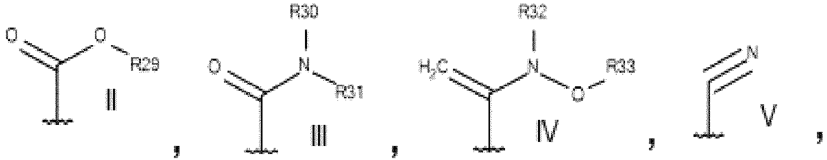 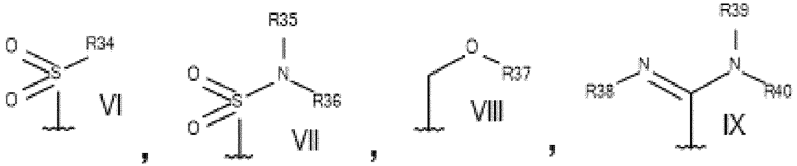 and
- R₇ and R₈ are independently chosen from the list comprising Aryl, subst. Aryl, Heteroaryl, subst. Heteroaryl,
whereat R₉ through R₄₀ are independently chosen from the list comprising H, F, Cl, Br, OH, phenyl, NH₂, NO₂, alkyl, branced alkyl, cycloalkyl, aryl, heteroaryl, substituted alkyl, substituted branched alkyl, substituted cycloalkyl, substituted aryl, substituted heteroaryl, alkylaryl, alkylheteroaryl, nitrile, amidine, guanidine.

9. A medicament according to one or more of the preceding claims, **characterized in that** it contains supplementary substances, chosen from the list comprising Citric Acid, Sodium Benzoate, Polyacrylic Acid, Calcium Carbonate, Starch, Lactose Sorbitol, Sucrose, Cellulose, Magnesiumstearate, Dicalciumphosphate, Calciumsulfate, Talc, Mannitol, Ethyl-alcohol, Methylcellulose, Polyvinyl Alcohol, denaturated Gelatine, Sodium-Carboxymethylstarch, natural and/or synthetic Gum like e.g. Arabic Gum, Carob Gum, Karaya Gum, Guar Gum, Tragacanth Gum, Agar, Cellulose derivatives like Methylcellulose, Sodium-Carboxymethylcellulose, microcrystalline Cellulose, Alginate, Alumina, Bentonite, Sugar, Starch from Grain, Rice or Potato, natural Gum like Acacia Gum, Gelatin, Traganth, Alginic Acid, Sodium Alginate, Ammonium-Calcium-Alginate, Methylcellulose, Cera, Sodium-Carboxymethylcellulose, Hydroxypropylmethylcellulose, Polyethylenglycole, Polyvinylpyrrolidone, inorganic compounds like Magnesium-Aluminum-Silicates, Boracic Acid, Stearates e.g. Magnesiumstearat, Calciumstearat, Potassiumstearat, Stearic Acid, high-melting Cera, Sodium-Chloride, Sodium-Benzoate, Sodium-Acetate, Sodium-Oleate, Polyethylenglycol, Amino-Acids like Leucine, Triglycerides of saturated Fatty Acids from plants, e.g. Triglycerin-Diisostearate, Esters of long-chain acids, e.g. Oleyl-Oleate, Isopropylmyristate, Isopropylpalmitate, 2-Ethylhexylpalmitate, Isooctylstearate, Isopropylstearate, Lauric Acid-Hexylester, Di-n-butyladipate, long-chain alkoholes, e.g. Hexylalcohol, Octylalkohol, Decylalkohol, Oleylalkohol, 2-Octyldodecanol, 2-Hexyldecanol, 2-Octyldecanol.

10. A medicament according to one or more of the preceding claims, **characterized in that** it contains the effective amount of Silvestrol and/or Episilvestrol and/or derivatives of Silvestrol or Episilvestrol and/or analoga of Silvestrol or Episilvestrol in protonated or deprotonated form, i.e. as salt.

11. Usage of a medicament according to one or more of the preceding claims, **characterized in that** it is applied in a preventive manner for the treatment of humans and/or mammals.
